# EUROPEAN PATENT SPECIFICATION

(11) **EP 1 366 998 B1**
(45) Date of publication and mention of the grant of the patent: **04.01.2006**
(21) Application number: 03252853.1
(22) Date of filing: 07.05.2003
(51) Int. Cl.: B65D 23/14

(54) **A system, method, and apparatuses for maintaining, tracking, transporting and identifying the integrity of a disposable specimen container with a re-usable transponder**
System, Verfahren und Vorrichtungen zur Aufrechterhaltung, Verfolgung, Förderung und Identifizierung der Integrität eines Einweg-Probenbehälters mit einem wiederverwendbaren Transponder
Système, procédé et dispositifs de préservation, suivi, transport et identification de l'intégrité d'un récipient à jeter comportant un transpondeur reutilisable

(30) Priority: 07.05.2002 US 140698
(43) Date of publication of application: 03.12.2003
(73) Proprietor: Capitol Plastic Products, LLC, Amsterdam, New York 12010 (US)
(72) Inventor: Abrams, Robert, Albany, NY 12203 (US); Giraud, Jean-Pierre, 75012 Paris12203 (FR)
(74) Representative: Stevens, Ian Edward

(56) References cited:
- WO-A-01/79988
- WO-A-01/81823
- CA-A- 2 204 207
- US-A- 5 133 470
- US-A- 5 660 301

## Description

### STATEMENT OF RELATED APPLICATIONS

This application claims the benefit of U.S. Serial No. 10/140,698 filed May 7, 2002 which is a continuation-in-part of pending U.S. patent application 09/947,596 filed September 5, 2001, which application claims the benefit of the filing date of provisional U.S. patent application no. 60/229,917, filed September 5,2000, abandoned.

### FIELD OF THE INVENTION

The present invention is directed to the system, method, and apparatuses for maintaining, tracking, and identifying the integrity of a disposable specimen container with a re-usable transponder.

### BACKGROUND OF THE INVENTION

Radio frequency identification (RFID) tags and radio frequency identification tag systems are used for identification and/or tracking of equipment or inventory such as pallets, trucks, dollies or boxes or even the whereabouts of some animals, such as livestock in certain situations. These RFID systems are radio communication systems in which communications is provided between a radio transceiver, or interrogator, and a number of small, identifying labels or tags. These tags are read while in the radiation pattern or field of the interrogator, which may be connected to a computer-based tracking system. The intent of an RFID system is to provide a reliable and secure architecture that meets a predetermined performance requirement, while minimizing the cost of the interrogator and the tags.

Conventionally, in the operation of RFID systems, the interrogator transmits to the tags using modulated radio signals, and the tags respond by transmitting modulated radio signals back to the interrogator. Specifically, the interrogator first transmits an amplitude-modulated signal to the tag. Next, the interrogator transmits a continuous-wave (CW) radio signal to the tag. The tag then modulates the CW signal using modulated back scattering (MBS) wherein the antenna is electrically switched, by the tag's modulating signal, from being an absorber of radio frequency (RF) radiation to being a reflector of RF radiation; thereby encoding the tag's information onto the CW radio signal. The interrogator demodulates the incoming modulated radio signal and decodes the tag's information message. A radio frequency identification tag system conveniently provides for reading the information from the radio frequency identification tag at a small distance using radio frequency (RF) data transmission technology. Typically, the user simply holds or places the radio frequency identification tag near a base station that transmits an excitation signal to the radio frequency identification tag powering circuitry contained on the radio frequency identification tag The circuitry, responsive to the excitation signal, communicates the stored information from the radio frequency identification tag to the base station, which receives and decodes the information. In general, radio frequency identification tags are capable of retaining and, in operation, transmitting a substantial amount of information-sufficient information to uniquely identify individuals, packages, inventory and the like.

In one application that is relevant to the present invention, specimen containers (e.g. vials) are used conventionally in the dairy and the drug testing industries. In such industries, a unique specimen sample (e.g. milk in the dairy industry, and blood or urine in the drug testing industry) is maintained in the vial. The unique specimen must be identified and tracked. In some applications, the specimens are identified and tracked by a unique bar code. Bar codes are typically located using a hand held optical scanner. Such bar code labeling systems utilize a light beam emitted from the scanner to "read" the bar code label. These systems require a direct line of sight between the scanner and the bar-code label, thus greatly limiting their utility.

In addition, the specimen containers may be provided with access that can be gained only by producing visible evidence that the container has been opened whether by accident or on purpose (e.g. use of tape or seal). Such a container is useful in the transportation and storage of liquid specimens for example, to ensure the integrity, of the specimen. The integrity of the specimen in the vial is becoming increasingly important in the dairy industry and for drug testing. It is important to ensure the so-called "guaranteed chain of custody" of the container contents by providing a "tamper-evident" seal to the vial - to protect from being opened by unauthorized personnel who might tamper with the contents.

### SUMMARY OF THE INVENTION

According to the invention, there is provided a container as defined in the appended claims 1 and 6. Preferred features of the container are set out in the dependent claims.

The present invention is a vial is provided with structure to house and retain an RFID tag. In yet another embodiment, the invention is a vial and RFID tag combination. The vial is intended for a single use, and then is disposed. The RFID tags are removed from the vial and reused as often as is possible.

The vial of the present invention can be, in its structure and design, a conventional vial with respect to its intended use, which for example could be a vial for holding a milk sample or a vial for holding a urine specimen, which then is provided with the structural attributes disclosed herein that enable it to house and retain an RFID tag. The vials of the present invention may be those vials disclosed in U.S. Patent nos. 5,012,941 and 5,133,470. These vial constructions would then be modified as disclosed herein in order to have the features that would allow the vials to receive and retain an RFID tag. Also, the vials may be provided with a breakaway tab that breaks off the first time the vial is opened.

In one embodiment, the vial comprises a container portion having a bottom wall, an internal cavity, and sidewall. In one embodiment, the bottom wall is offset from the bottom of the sidewall. This arrangement defines a rim that extends from sidewall bottom to bottom wall.

A retaining ring, having a perimeter that fits within the perimeter of the rim, is joined to the bottom wall and extends in the direction of the sidewall bottom. The retaining ring has regions of a first smaller height dimension and lugs, which are regions of a second greater height dimension. Further, spacer elements are positioned at several locations around the inner wall of the retaining ring. The spacer elements may be positioned along the retaining ring in the regions of the first smaller height dimension, approximately halfway between the lugs. A depression is provided in the center of the bottom wall.

To secure the RFID tag, it is placed within the retaining ring. The lugs are then reconfigured, that is, moved out of the substantially vertical state to a state, that is at least partially horizontal, in which the lugs extend over the tag in order to secure it in place. Any technique that imparts sufficient energy to the plastic vial in order to soften the lugs, so that they can be bent over the RFID tag, can be employed. Ultrasonic energy may be employed for this task. Alternatively, energy from a heat source may also be applied.

The vials can be constructed of plastic materials such as polypropylene and polyethylene. The plastic materials that are employed should not be adversely affected by a sterilizing dose of gamma radiation.

In yet another embodiment, a notch is cut out of the rim. The notch is employed to properly orient the vial during opening.

There is disclosed herein a puck that is used to escort the vial assembly along the conveyor system. The puck has a cavity positioned within the surrounding sidewalls. The cavity is sized maintain the vial in an upright position. That is, it is dimensioned so that the outer surface of the vial contacts the inner wall of the cavity and/or is in close proximity thereto. In a further embodiment, the puck is provided with a bottom surface and cavity-defining sidewalls that extend upward from the bottom surface. The puck may be provided with openings positioned in the sidewalls, in the bottom surface, or in both locations. Furthermore, the puck may be provided with a structural attribute that, in cooperation with a structural attribute of the vial assembly, aligns and orients the vial, so that the vial is properly positioned at the time it visits the stations along the path where testing is conducted. For example, a notch can be located at the bottom of the vial, and the puck can be provided with a feature that mates with the notch. This arrangement can be used to insure that the vial is properly aligned for its visit to the lid opening station, thereby optimizing the number of vials successfully opened by automated equipment.

### BRIEF DESCRIPTION OF THE DRAWINGS

Figure 1 is a perspective view of an embodiment of the present invention, with the RFID tag present.
Figure 2 is a perspective view of an embodiment of the present invention, with the RFID tag absent.
Figure 3 is a side elevational view of an embodiment of a puck;
Figure 4 is side elevational view showing a vial retained in a puck;
Figure 5 is cross sectional views of the walls of the vial and puck while the former is retained in the latter;
Figure 6 is a top view showing, in cross section, the bottom of the vial and puck while the former is retained in the latter;
Figure 7 is a top view showing, in cross section, the bottom and top of the vial and puck while the former is retained in the latter;
Figure 8 is a perspective view showing the engagement between the puck and vial; and
Figure 9 is a perspective view showing the manner in which the lugs can be shapen over the RFID tag.

### DETAILED DESCRIPTION OF THE INVENTION

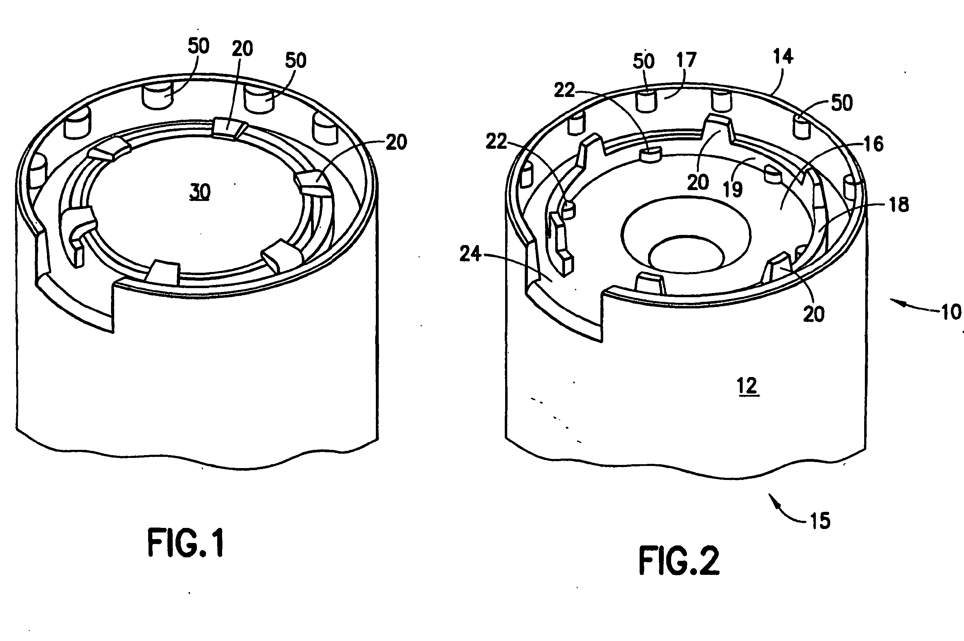

The vial of the present invention can be, in its structure and design, a conventional vial with respect to its intended use, which for example could be a vial for holding a milk sample or a vial for holding a urine specimen, which then is provided with the structural attributes disclosed herein that enable it to retain an RFID tag. The vials of the present invention may be those vials disclosed in U.S. Patent nos. 5,012,941 and 5,133,470. These vial constructions would then be modified as disclosed herein in order to have the features that would allow the vials to receive and retain an RFID tag. Also, as disclosed in the patents incorporated herein by reference, the vials may be provided with a breakaway tab that breaks off the first time the vial is opened. As shown in Figures 1 and 2, the vial comprises a container portion 10 having a bottom wall 16, an internal cavity 15, and sidewall 12. The bottom wall 16 is offset from the bottom 14 of the sidewall 12. This arrangement defines a rim 17 having a length dimension that extends from sidewall bottom 14 to bottom wall 16.

A retaining ring 18 has a perimeter positioned within the perimeter of the rim 17. The retaining ring 18 is joined to the bottom wall 16 and extends for at least a portion of the length dimension of the rim 17, in the direction of sidewall bottom 14. The retaining ring has regions of a first smaller length dimension 19 and lugs 20, which are regions of a second greater length dimension. Spacer elements 22 are positioned at several locations around the inner wall 23 of the retaining ring. The spacer elements may be positioned along the retaining ring 20 in the regions of the first smaller height dimension 19, approximately halfway between the lugs 20. A depression is provided in the center of the bottom wall 16.

The rim is further provided with a number of ribs 50, that extend inward. The ribs are positioned around the perimeter of the rim 17.

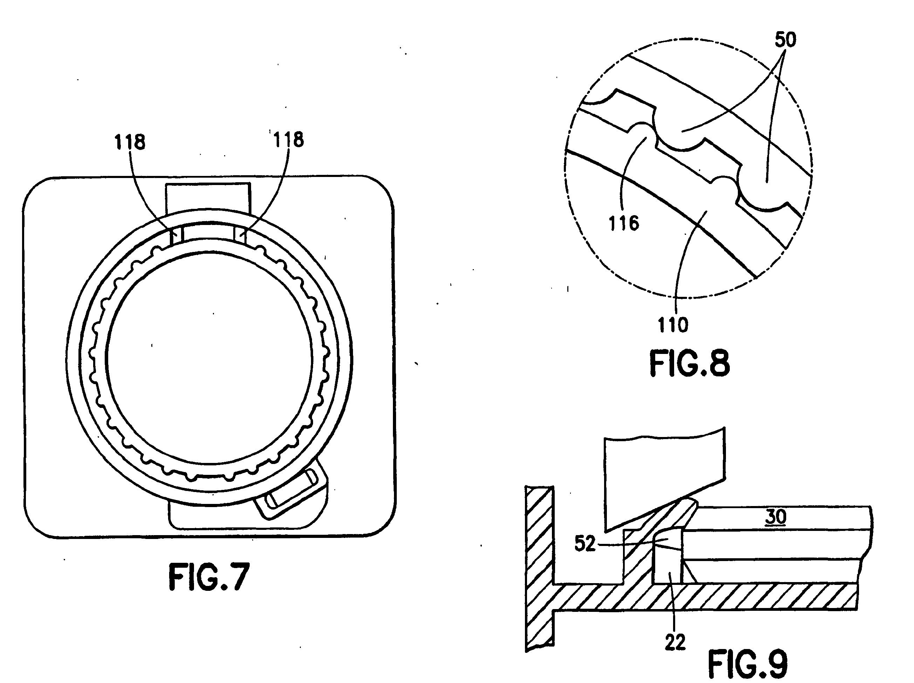

To secure the RFID tag 30, it is placed within the retaining ring. The lu gs 20 are then bent down and in over the tag to secure it in place. Any technique that imparts sufficient energy to the plastic vial in order to soften the lugs, so that they can be bent over the RFID tag, can be employed. In one example, ultrasonic energy may be employed for this task. Figure 9 demonstrates an arrangement by which the lugs 20 can be reconfigured in order to extend over the RFID tag and retain it in place. Ultrasonic devices that can be used to reshape the lugs are available from Branson Ultrasonics Corporation, Danbury CT USA.

Alternatively, energy from a heat source may also be applied. A mechanism that applies pressure to the lugs when they are in the softened state can be employed to effect the bending of the lugs. The mechanism may have a bar or plate or the like that effects bending from the original, substantially vertical state of the lugs, to the final bent state in which the lugs are at least partially horizontal. This component may be employed to retain the lugs in the bent state until after the plastic cools, at which time the lugs will remain in the bent state after pressure is removed.

The vials can be constructed of plastic materials such as polypropylene and polyethylene. The plastic materials that are employed should not be adversely affected by a sterilizing dose of gamma radiation.

In yet another embodiment, a notch 24 is cut out of the rim 17. The automated equipment that opens the vials prior to drawing a sample employs the notch in order to properly orient the vial, to insure that the vial is correctly opened. The vial should have a diameter large that is large enough so that the vial can hold an RFID tag (e.g. a 22 mm diameter tag, a 17mm square tag). Consequently, these dimensions set the size required for the retaining ring. While the arrangement shown in the Figures shows a circular tag, it should be readily understood that the person of skill in the art could modify the embodiments shown herein to provide the necessary adaptations for any other shaped tag (e.g. square tag).

The lugs that hold the RFID tags should be designed to be strong enough to withstand one complete use cycle. Merely by way of example, with regard to a milk sample, this includes travel to and from and processing at the following locations: the tag insertion station, the transport rack, the milk tanker cool box, the milk tanker RFID read/write station, the transport racks, the laboratory automation, and the tag removal station.

One advantage of the structure disclosed herein is that the lugs provide evidence of tampering, or the lack thereof. That is, observation of the lugs will provide visual indication on whether they have been tampered with.

As detailed below, the vials of the present invention can be formed in the mold. The retaining ring, spacers, rim and other features described above can also be formed in the mold by these same molding techniques.

In another aspect, the present invention generally relates to a method of maintaining, tracking and identifying the integrity of a specimen container using a disposable specimen container and a reusable RFID tag.

Suitable RFID devices include a read-only or a read/write transponder. For a read/write RFID device, the transponder acts as a both a storage device and a display device. Examples of RFID devices suitable for the present invention include: (a) the "RI-TRP," "RI-101," "RI-102," and "RI-103" models from Texas Instruments; and (b) the "GemWave Ario" and the "GemFly" models from Gemplus. The specific transponder may be chosen based on the specific application including: (a) the size and shape of the container and thus, the maximum surface area that is available for the transponder, (b) the environment (humidity, hazards and degree of special handling); (c) the need to re-use the transponder, (d) the memory capacity; (e) the size of the antenna; and (f) the cost. It is understood that the RFID device of the present invention includes any equivalent device that has the capability of both reading and writing such as the category referred to as "transponders."

For the dairy industry, the following is one embodiment of the method of the present invention:
(a) a disposable vial and RFID tag combination that are assembled as described above;
(b) information including the date and a unique identification of the vial is written to the RFID device attached to the individual specimen vial;
(c) the vials are sent to the dairy (and/or given to a tank driver);
(d) when the milk is pumped from a specific tank at the dairy to the tanker truck, a milk sample is taken (either automatically or manually) and is put in the specimen vial;
(e) information including the specific dairy, the specific dairy tank, the time, the day and/or the temperature of the milk are written to the RFID device attached to the specimen vial;
(f) the vial is then stored in an environment to maintain its integrity (e.g. insulated container, refrigerator unit);
(g) the vial(s) are sent to a laboratory for analysis;
(h) at the laboratory, the vials are inventoried by scanning the RFIDs attached to the individual vials;
(i) at the laboratory, information including the routing (e.g. test required such as fat/protein/bacteria/antibiotics analysis) of the sample is written to the RFID attached to the individual specimen vial; and
(j) the RFID device is separated from the vial so that the RFID device may be re-used and the corresponding vial is ground-up so that the plastic may be recycled.

Steps (a) and (b) can be effected by an automated process that is practiced at the site where the RFID tag is joined to the vial. Steps (d) and (e) take place at the dairy. The information mentioned in step (e) may be written to the tag by employing a hand held device, after first inputting the necessary information on a keypad or other device. Steps (h), (i), and possibly (j) occur at the testing laboratory. Steps (h) and (i) may occur along the automated assembly line, and information read from the vials can be employed to determine and assign the ultimate destination of the vial, such as the test station for nutritional analysis. Alternatively, at a given location on the conveyor, information may be written to the tag, which is used further on down the conveyor to assess and determine the ultimate destination of the vial. A series of RFID reader and writer devices are provided on the conveyor system in order to read and write information from and to the RFID tags.

While step (j) can occur at the laboratory, it may also occur at a remote location, which may include the location where steps (a) and (b) take place. Removal of the RFID tags may be an automated process step.

In a more specific embodiment of the above-described method, a hand-held scanner may be used to either write information to and/or read information from the RFID device. For example, the tank driver and/or the dairy may use the hand-held scanner to read/write information including: (a) the date and a unique identification to the RFID device attached to the individual specimen vial; and (b) when the milk is pumped from a specific tank at the dairy to the tanker truck, the specific dairy, the specific dairy tank, and/or the temperature of the milk to the RFID device attached to the specimen vial. In another example, the laboratory personnel may use the hand-held scanner to read/write information including: (a) an inventory of the individual vials by reading the unique identification corresponding to the vial; and/or (b) routing information (e.g. test required such as fat/protein/bacteria/antibiotics analysis) of the sample by writing to the RFID device attached to the individual specimen vial. In another embodiment, the RFID device attached to the vial may be scanned without requiring a direct line of sight between the scanner and the RFID device. In this way, the vials contained within the larger containers do not need to be taken out and individually scanned.

For the drug testing industry, the following is one embodiment of the method of the present invention:
(a) a disposable vial and RFID tag combination that are assembled as described above;
(b) information including the date and a unique identification of the vial is written to the RFID device attached to the individual specimen vial;
(c) the vials are sent to the office (e.g. physicians office, testing laboratory) where the patients urine or blood specimen is obtained;
(d) when the patient's blood or urine specimen is obtained, the specimen is put in the specimen vial;
(e) information including the individual's identification, the time, the day and/or additional office information are written to the RFID device attached to the specimen vial;
(f) the vial(s) are sent to a laboratory for analysis;
(g) at the laboratory, the vials are inventoried by scanning the RFIDs attached to the individual vials;
(b) at the laboratory, information including the routing (e.g. test required such as the type of drug to be tested for) of the sample is written to the RFID attached to the individual specimen vial; and
(i) after all testing is complete and the specimen is no longer needed, the RFID device is separated from the vial so that the RFID device may be re-used and the corresponding vial is ground-up so that the plastic may be recycled.

In yet a further embodiment of the present invention, the routing step may be automated so a conveyor-like system is designed where the vials are automatically routed to the proper station based on a scanner reading the individual RFID devices attached to each vial.

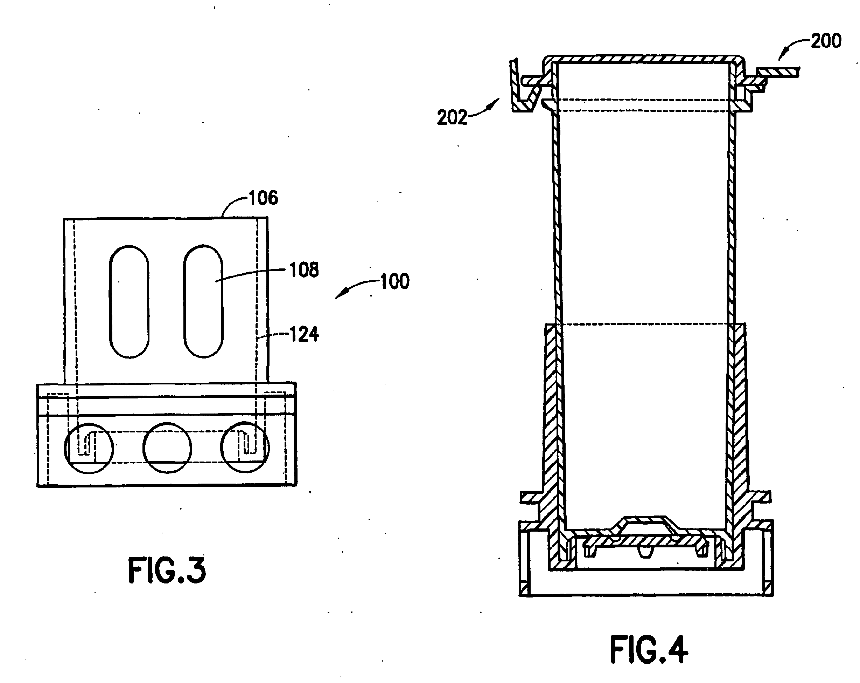

In yet another embodiment, the invention is a puck that escorts the vial around the assembly lines described above for milk testing and drug testing operations. Figures 3-8 show a puck 100, both alone and with a vial 10 disposed inside of it. Figure 3 shows the exterior of the puck, having a base portion 102 of greater cross section than the elevated portion 104 that rises above it. The base portion and the elevated portion form the sidewalls of a cavity 106 in which the vial 10 resides. Both the base portion and the elevated portion are provided with through-holes 108, that, among other things, permit water to freely circulate over the surface of the vial. In some instances, it may be desirable to flow warm or hot water over the vials while they reside in the pucks.

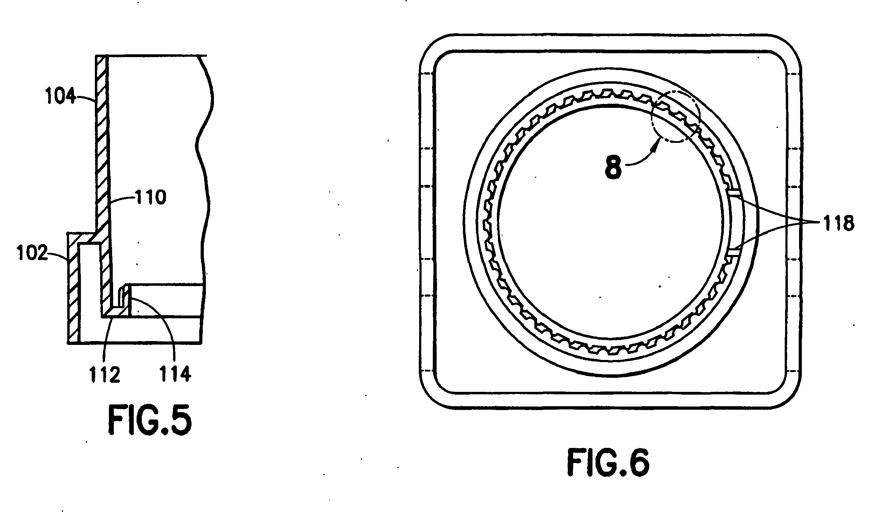

As shown in Figures 4 and 5, the bottom region 112 of puck sidewall 110 is provided with an upward-turned hook 114 that grasps the rim 17 of the vial 10. The bottom region 112 of puck sidewall 110 is further provided with a number of ribs 116. Ribs 116 are provided on the puck 100. The ribs 116 extend into the space between the bottom region 112 and the hook 114. The ribs 116 of the puck 100 are positioned around the bottom region, at the place where the hook 114 and bottom region 112 come together. When the vial 10 is placed within the puck 100, the ribs 116 of the puck 100 engage with ribs 50 on the rim 17 of the vial 10. The ribs 116 of the puck and the ribs 50 on the rim 17 of the vial 10 form a frictional engagement, which aids in retaining the vial in place.

As best seen in Figure 7, the puck is provided with bars 118 that are spaced apart from each other. The bars 118 are positioned to extend into the notch 24 on the vial 10 when the vial 10 is placed in the puck 100. Having the bars extend into the notch insure that the vial is properly oriented. The bars 118 engage the inner surface of the notch 24 that is provided in the rim 17 of the vial 10. The bars 118 orient the vial 10 so that it is properly aligned for certain procedures that are undertaken along the conveyor, such as the automated opening of the vial lid.

The pucks may be constructed of a strong durable material, such as a polyamide. In some embodiments, the polyamide may be metal-filled in order to modify the heat transfer properties of the polymer.

The automated opening of the lid may be accomplished in the following manner. As just described, the properly oriented vial will enter the lid opening station. A retaining element 200 (see Figure 4) will contact the hinge that joins the lid to the container portion of the vial, applying a downward force on the hinge. A book 202 will then approach the tab on the front portion of the lid and contact the tab on the underside thereof. The hook will then be moved upward, which applies an upward force to the lid and thereby opens the vial. The pucks facilitate the movement of the vial along conveyor systems, and where necessary, maintain the vial in an upright position. Examples of various stations along the conveyor, when the puck may aid the transport and orientation of the vial, include: (a) sample heating stations; (b) mixing stations; (c) maintaining orientation of the vial for through a station or stations for automated venting, opening and closing of the lid.

The pucks are sized at their base with dimensions sufficient to confer stability to the vial, yet small enough to minimize the space requirement in the buffer and storage areas. That is, the puck remains stable while it is being moved around by the conveyor system.

The puck can be any shape, bowever, to maintain orientation, it may be preferable to provide shapes that will avoid rotational movement while moving along the conveyor, such as non-circular shapes. Squares may be well suited for the shape of the puck.

Vials situated in pucks will arrive at the tag removal station. It may be desirable to remove the tags while the vial is still in the puck. To this end, a hole in the bottom of the puck may be provided. The hole should be large enough to accommodate a tag removal tool and permit the removal of the tag. As shown in Figure 9, a gap 52 is present between the spacer and the lug. During removal of the RFID tag, the cutting blade that is employed to cut the lugs will move completely through the lugs and extend into the gap, insuring that the lugs are completely severed. During the cutting operation, the vial may be partially rotated, possibly within the range of 10°-15°, or as required by the dimensions of the lugs, to aid in the severing operation.

A sidewall of approximately 1 or 2 mm around the circumference of the base should be sufficient to locate and retain the vial.

At various points in the automated system, the samples (and pucks) will be heated to 40°C in sample heating stations. The sample heating stations may move the pucks along X-Y coordinates in a heating station, while the samples are sprayed with hot water. Likewise, since the vials circulate in a milk-testing environment, where there is bound to be spillage, it may be desirable to clean them in a dedicated cleaning station - using hot water and optionally, an anti-bacterial agent. Providing openings in the puck will maximize contact between the fluid and the vial.

In a further embodiment of the RFID device, radio frequency identification tag is formed by directly joining a radio frequency identification tag circuit chip ("circuit chip") to an article having an integrally formed antenna. Article may be a substrate formed from a sheet of material. The substrate may be the specimen container. Moreover, the substrate material may be any suitable material for the particular application such as paper, plastic (including polyester and metalized polyester material), synthetic paper, reinforced paper, cardboard, coated cardboard and the like.

In one embodiment, the RFID tag is attached to the container, which identifies the container and thus the customer. For example, this tag may be secured to any part of the container including the lid, the bottom, the side or the top of the container. As such, the vial may be specifically designed to accommodate the RFID device. Such a tag may include a relatively flat or thin coil connected to an integrated circuit (IC) disposed within the confines of the coil. Thus, the coil of RFID tag is disposed substantially in a horizontal plane within the lid.

The apparatus and process of the present invention may be used with a variety of bodies including bottles, vials, spouts or any other containers. Although the examples describe a vial, the invention covers any type of container that may be used to transport specimen samples. The invention is described in the description with respect to a vial.

In another embodiment, the vial may be cylindrical in shape with an integrally formed bottom. A cap may be provided which is adapted to seal the vial closed with a substantially hermetic seal. The cap may be integrally connected to the vial with a small flange. The vial and cap may be injection molded in the mold from a thermoplastic material. Examples of processes of making such vial and of designs for such vials are disclosed in U.S. Patents Nos. 4,812,116, 4,783,056, 5,723,085, and 6,303,064.

In a further embodiment, vials of the type to which the present invention relates are generally injection-molded plastic vials that have caps adapted to seal the vial closed with a substantially hermetic seal. The cap may or may not be integrally connected to the vial, but is preferably joined thereto with a small flange. It is important to maintain the sterility of the interior of the vial prior to use. Accordingly, in order to maintain the sterility of the interior of the vial the cap must be closed onto the vial while the vial is in an aseptic environment.

In a further embodiment of the present invention, the vial is designed so that the RFID device is sufficiently secured to the vial so that the RFID device remains attached to the vial during regular shipping and handling. Such a securing device may include, but are not limited to, a clip-on system, a slotted system, and snap-on system. At the same time, the securing device is designed so that, after the specimen has been tested, the RFID device may be intentionally removed from the vial with out incurring damage to the RFID device. In this way, the RFID device may be re-used while the corresponding vial is ground-up and the plastic material may be recycled. The present invention takes advantage of the ability to dispose of the vial after each use so as to maintain integrity and sterility of the specimen sample while, at the same time, to re-use the RFID device. Consequently, the present invention is environmentally "green" - the vial may be recycled and the plastic re-used and the RFID device may be continually re-used. In one embodiment, an automatic system may be designed where the RFID is automatically removed from a used vial and is inserted in a new vial while the used vial is ground-up and prepared for recycling. One process of automatically recycling the vial is disclosed in U.S. Patent No. 5,979,804.

In another aspect of the present invention, the vial may be a tamper-proof container and cap for indicating whether the container has been opened during transport to or from a specimen-receiving site. For example, one or more destructible connections are provided between the container and cap, that connection including one or more destructible members which hold the cap in a closed condition whereby the cap can be opened only in response to the destruction of the destructible member. Accordingly, an opening of the closed container during transport thereof to a specimen-receiving site (e.g. a laboratory) is evident from a destruction of the destructible member. One embodiment of such a tamper-proof design is described in U.S. Patent No. 5,012,941.

In yet another aspect of the present invention, the device that secures the RFID device to the vial may be a tamper-proof design for indicating whether the RFID device has been either replaced with another RFID device or has been tampered with during transport to or from a specimen-receiving site and/or during handling. For example, one or more destructible connections may be provided between the RFID device and container, that connection including one or more destructible members which hold the RFID device to the container whereby the RFID device can be removed from the container only in response to the destruction of the destructible member. Accordingly, tampering with the RFID device during transport and/or handling thereof to a specimen-receiving site (e.g. a laboratory) is evident from a destruction of the destructible member.

## Claims

1. A container comprising:
a disposable plastic vial;
a cap;
a recessed portion located on a bottom side of the container (10);
wherein the recessed portion has lugs (20) that are fixable into a retaining position for retaining an RFID tag in place; and
said lugs can be softened by the action of a heating source and fixed into the retaining position by the action of pressure in the softened state.

2. The container of claim 1, further comprising a notch (24) located in the bottom side.

3. The container of claim 1 comprising an RFID tag retained in place by the lugs.

4. The container of claim 1 wherein the recessed portion is offset from a bottom end of the container by a rim (17).

5. The container of claim 4, further comprising a notch (24) located in the rim (17).

6. A container comprising a bottom surface recessed from a bottom end of a container sidewall, the bottom surface comprised of:
a ring (18) depending from the bottom surface in a direction away from the bottom surface to a first height dimension;
a plurality of lugs (20) extending from the ring in a direction further away from the bottom surface to a second height dimension greater than the first height dimension;
a plurality of spacers (22) positioned on an interior side of the ring;
wherein a space is defined interior the spacers in which a RFID tag can be positioned, and wherein the lugs can be fixed into a position in which they extend over the tag when the tag is positioned in the space.

7. The container of claim 6 wherein the bottom surface is offset from the bottom end of the container by a rim (17).

8. The container of claim 6, further comprising a notch (24) located in the bottom side.

9. The container of claim 8, wherein the notch (24) is located in the rim (17).

10. The container of claim 6 wherein the rim (17) is provided with a plurality of ribs (50).

11. The container of claim 1 wherein the container and cap are tamper-proof for indicating whether the container has been opened, and comprising:
a destructible projection extending from one toward the other of said container and
cap, said projection including a stem integrally molded with said one of said
container and cap and at least one laterally extending flange at a free end of said
stem, said stem including a single weakened point of reduced cross section disposed at an end of said stem opposite said free end thereof, a cross piece projecting laterally from said stem at a location intermediate said free end and said
weakened point, said cross piece being coplanar with said stem and said flange,
an elongated slot formed in the other of said container and cap and receiving said
free end of said projection, said flange extending laterally beyond an edge of said
slot such that said cap can be opened only in response to breakage of said projection at said weakened point, said slot being situated between said flange and
cross piece to prevent separation of said projection from said other of said container and cap after said projection has been broken,
said container including a channel extending circumferentially around an outer
surface thereof, said channel being open toward said cap, said channel receiving a peripheral edge of said cap when said cap is closed.

12. The container of claim 11 comprising an RFID tag retained in place by the lugs.

13. The container of claim 11 wherein the recessed portion is offset from a bottom end of the container by a rim.

14. The container of claim 12, further comprising a notch located in the rim.

15. The container of claim 1 wherein the container and cap are tamper-proof for indicating whether the container has been opened, and comprising:
a destructible connection between said container and cap including a destructible
member holding said cap in a closed condition such that said cap can be opened
only in response to destruction of said destructible member, said container
including an annular rim forming a mouth of said container, and an upwardly open
annular channel extending circumferentially around an outer surface of said
container at a location below said rim,
said cap including:
a recess for receiving said rim to close said mouth,
a peripheral edge received in said channel when said cap is closed, and
an annular seal member disposed within said recess at a location above said
peripheral edge, said annular seal member forming a downwardly open gap in
which said rim is received to form a seal.

16. The container of claim 15 comprising an RFID tag retained in place by the lugs.

17. The container of claim 15 wherein the recessed portion is offset from a bottom end of the container by a rim.

18. The container of claim 17, further comprising a notch located in the rim.

19. The container of claim 1 wherein the container and cap are tamper-proof for indicating whether the container has been opened, and comprising:
a destructible projection extending from one toward the other of said container and
cap, said projection including a stem integrally molded with said one of said
container and cap and at least one laterally extending flange at a free end of said
stem, said stem including a single weakened point of reduced cross section disposed at an end of said stem opposite said free end thereof, a cross piece
projecting laterally from said stem at a location intermediate said free end and said
weakened point, said cross piece being coplanar with said stem and said flange,
an elongated slot formed in the other of said container and cap and receiving said
free end of said projection. said flange extending laterally beyond an edge of said
slot such that said cap can be opened only in response to breakage of said projection at said weakened point, said slot being situated between said flange and cross piece to prevent separation of said projection from said other of said container and cap after said projection has been broken, said cap being molded of one-piece with said container, and said cap and container
being molded of plastic.

20. The container of claim 19 comprising an RFID tag retained in place by the lugs.

21. The container of claim 19 wherein the recessed portion is offset from a bottom end of the container by a rim.

22. The container of claim 21, further comprising a notch located in the rim.

23. The container of claim 1 wherein the container and cap are tamper-proof for indicating whether the container has been opened, and comprising:
a destructible projection extending from one toward the other of said container and
cap, said projection including a stem integrally molded with said one of said
container and cap and at least one laterally extending flange at a free end of said
stem, said stem including a single weakened point of reduced cross section disposed at an end of said stem opposite said free end thereof. a cross piece projecting laterally from said stem at a location intermediate said free end and said
weakened point, said cross piece being coplanar with said stem and said flange,
an elongated slot formed in the other of said container and cap and receiving said
free end of said projection, said flange extending laterally beyond an edge of said
slot such that said cap can be opened only in response to breakage of said projection at said weakened point, said slot being situated between said flange and
cross piece to prevent separation of said projection from said other of said container and cap after said projection has been broken,
said container and cap including a second destructible member adapted for one-way
passage through a hole in another of said container and cap.

24. The container of claim 23 comprising an RFID tag retained in place by the lugs.

25. The container of claim 23 wherein the recessed portion is offset from a bottom end of the container by a rim.

26. The container of claim 25, further comprising a notch located in the rim.

27. The container of claim 1 wherein the container and cap are tamper-proof,
said container including a first flange having a first through-hole, said cap
including a second flange having a second through hole,
said second flange arranged to substantially overlie said first flange when said
cap is inserted onto a rim of said container, one of said container and cap including
an integral strip extending from a location adjacent said through-holes, said strip
being of sufficient flexibility and length to enable said strip to be inserted through
said through-holes while remaining attached to said one of said container and cap,
said strip including portions configured for one-way passage through said through holes,
wherein the container further is provided with a recessed portion located on
a bottom side of the container, the recessed portion having lugs that are fixable into
a retaining position for retaining an RFID tag in place.

28. The container of claim 27 comprising an RFID tag retained in place by the lugs.

29. The container of claim 27 wherein the recessed portion is offset from a bottom end of the container by a rim.

30. The container of claim 29, further comprising a notch located in the rim.

31. The container of claim 1 wherein the container and cap are tamper-proof comprising a container and a cap of integrally molded one piece construction and including a hinge therebetween, said container including a first flange having a first through-hole, said cap including a second
flange arranged to overlie said first flange when said cap is inserted onto a rim of
said container, said flange of one of said container and cap including a strip
integrally molded in one piece therewith, said strip being sufficiently flexible and
of a length sufficient to enable said strip to be inserted through said holes while
remaining attached to said last-named flange, said strip including portions
configured for one-way passage through said through-holes whereby removal of
said strip is possible only by permanent destruction thereof, said second flange
defining a thumb tab facilitating opening of said cap.

32. The container of claim 31 comprising an RFID tag retained in place by the lugs.

33. The container of claim 31 wherein the recessed portion is offset from a bottom end of the container by a rim.

34. The container of claim 33, further comprising a notch located in the rim.

## Patentansprüche

1. Behälter, umfassend
eine Einweg-Kunststoffphiole;
eine Kappe;
einen vertieften Abschnitt, der sich an einer Unterseite des Behälters (10) befindet,
wobei der vertiefte Abschnitt Ansätze (20) aufweist, die in eine Rückhaltestellung fixiert werden können, um eine Funkfrequenzkennungsmarke an ihrer Stelle zurückzuhalten; und
wobei die Ansätze durch die Wirkung einer Erhitzungsquelle erweicht werden können und durch die Wirkung von Druck im erweichten Zustand in die Rückhaltestellung fixiert werden können.

2. Behälter nach Anspruch 1, ferner umfassend eine Einkerbung (24), die sich in der Unterseite befindet.

3. Behälter nach Anspruch 1, umfassend eine Funkfrequenzkennungsmarke, die durch die Ansätze an ihrer Stelle zurückgehalten wird.

4. Behälter nach Anspruch 1, wobei der vertiefte Abschnitt durch einen Rand (17) von einem unteren Ende des Behälters versetzt ist.

5. Behälter nach Anspruch 4, ferner umfassend eine Einkerbung (24) im Rand (17).

6. Behälter, umfassend eine Bodenfläche, die von einem unteren Ende einer Behälterseitenwand vertieft ist,
wobei die Bodenfläche Folgendes umfasst:
einen Ring (18), der in einer von der Bodenfläche wegführenden Richtung bis zu einer ersten Höhenabmessung von der Bodenfläche hängt;
mehrere Ansätze (20), die sich in einer weiter von der Bodenfläche wegführenden Richtung bis zu einer zweiten Höhenabmessung, die größer als die erste Höhenabmessung ist, vom Ring erstrecken;
mehrere Distanzstücke (22), die an einer Innenseite des Rings angeordnet sind;
wobei innerhalb der Distanzstücke ein Raum definiert ist, in dem eine Funkfrequenzkennungsmarke angeordnet werden kann, und wobei die Ansätze in eine Stellung fixiert werden können, in der sie sich über die Marke erstrecken, wenn die Marke im Raum angeordnet ist.

7. Behälter nach Anspruch 6, wobei die Bodenfläche durch einen Rand (17) vom unteren Ende des Behälters versetzt ist.

8. Behälter nach Anspruch 6, ferner umfassend eine Einkerbung (24), die sich in der Unterseite befindet.

9. Behälter nach Anspruch 8, wobei sich die Einkerbung (24) im Rand (17) befindet.

10. Behälter nach Anspruch 6, wobei der Rand (17) mit mehreren Rippen (50) versehen ist.

11. Behälter nach Anspruch 1, wobei der Behälter und die Kappe manipulationssicher sind, um anzuzeigen, ob der Behälter geöffnet worden ist, umfassend
einen zerstörbaren Vorsprung, der sich von einem aus dem Behälter und der Kappe zum anderen erstreckt,
wobei der Vorsprung einen Schaft beinhaltet, der einstückig mit einem aus dem Behälter und der Kappe geformt ist, und an einem freien Ende des Schafts zumindest einen sich seitlich erstreckenden Flansch beinhaltet, wobei der Schaft einen einzelnen geschwächten Punkt mit einem verringerten Querschnitt beinhaltet, der an einem Ende des Schafts gegenüber seinem freien Ende angeordnet ist, wobei sich ein Querstück an einer Stelle zwischen dem freien Ende und dem geschwächten Punkt seitlich vom Schaft erstreckt,
wobei das Querstück koplanar mit dem Schaft und dem Flansch ist,
einen länglichen Schlitz, der im anderen aus dem Behälter und der Kappe ausgebildet ist und das freie Ende des Vorsprungs aufnimmt, wobei sich der Flansch seitlich über einen Rand des Schlitzes hinaus erstreckt, so dass die Kappe nur als Reaktion auf einen Bruch des Vorsprungs am geschwächten Punkt geöffnet werden kann, wobei sich der Schlitz zwischen dem Flansch und dem Querstück befindet, um eine Trennung des Vorsprungs vom anderen aus dem Behälter und der Kappe zu verhindern, nachdem der Vorsprung zerbrochen wurde,
wobei der Behälter einen Kanal beinhaltet, der sich umfänglich um eine Außenfläche davon erstreckt, wobei der Kanal zur Kappe hin offen ist, wobei der Kanal einen peripheren Rand der Kappe aufnimmt, wenn die Kappe geschlossen ist.

12. Behälter nach Anspruch 11, umfassend eine Funkfrequenzkennungsmarke, die durch die Ansätze an ihrer Stelle zurückgehalten wird.

13. Behälter nach Anspruch 11, wobei der vertiefte Abschnitt durch einen Rand von einem unteren Ende des Behälters versetzt ist.

14. Behälter nach Anspruch 12, ferner umfassend eine Einkerbung, die sich im Rand befindet.

15. Behälter nach Anspruch 1, wobei der Behälter und die Kappe manipulationssicher sind, um anzuzeigen, ob der Behälter geöffnet worden ist, umfassend
eine zerstörbare Verbindung zwischen dem Behälter und der Kappe, die ein zerstörbares Element beinhaltet, das die Kappe in einem geschlossenen Zustand hält, so dass die Kappe nur als Reaktion auf die Zerstörung des zerstörbaren Elements geöffnet werden kann, wobei der Behälter einen ringförmigen Rand beinhaltet, der eine Öffnung des Behälters bildet, und einen aufwärts gerichtet offenen Kanal beinhaltet, der sich an einer Stelle unterhalb des Rands umfänglich um eine Außenfläche des Behälters erstreckt,
wobei die Kappe Folgendes beinhaltet:
eine Vertiefung zum Aufnahmen des Rands um die Öffnung zu schließen,
einen peripheren Rand, der im Kanal aufgenommen ist, wenn die Kappe geschlossen ist, und
ein ringförmiges Dichtungselement, das an einer Stelle über dem peripheren Rand in der Vertiefung angeordnet ist, wobei das ringförmige Dichtungselement eine abwärts gerichtet offene Lücke bildet, in der der Rand aufgenommen ist, um eine Dichtung zu bilden.

16. Behälter nach Anspruch 15, umfassend eine Funkfrequenzkennungsmarke, die durch die Ansätze an ihrer Stelle zurückgehalten wird.

17. Behälter nach Anspruch 15, wobei der vertiefte Abschnitt durch einen Rand von einem unteren Ende des Behälters versetzt ist.

18. Behälter nach Anspruch 17, ferner umfassend eine Einkerbung, die sich im Rand befindet.

19. Behälter nach Anspruch 1, wobei der Behälter und die Kappe manipulationssicher sind, um anzuzeigen, ob der Behälter geöffnet worden ist, umfassend einen zerstörbaren Vorsprung, der sich von einem aus dem Behälter und der Kappe zum anderen erstreckt,
wobei der Vorsprung einen Schaft beinhaltet, der einstückig mit einem aus dem Behälter und der Kappe geformt ist, und an einem freien Ende des Schafts zumindest einen sich seitlich erstreckenden Flansch beinhaltet, wobei der Schaft einen einzelnen geschwächten Punkt mit einem verringerten Querschnitt beinhaltet, der an einem Ende des Schafts gegenüber seinem freien Ende angeordnet ist, wobei sich ein Querstück an einer Stelle zwischen dem freien Ende und dem geschwächten Punkt seitlich vom Schaft erstreckt,
wobei das Querstück koplanar mit dem Schaft und dem Flansch ist,
einen länglichen Schlitz, der im anderen aus dem Behälter und der Kappe ausgebildet ist und das freie Ende des Vorsprungs aufnimmt, wobei sich der Flansch seitlich über einen Rand des Schlitzes hinaus erstreckt, so dass die Kappe nur als Reaktion auf einen Bruch des Vorsprungs am geschwächten Punkt geöffnet werden kann, wobei sich der Schlitz zwischen dem Flansch und dem Querstück befindet, um eine Trennung des Vorsprungs vom anderen aus dem Behälter und der Kappe zu verhindern, nachdem der Vorsprung zerbrochen wurde,
wobei die Kappe einteilig mit dem Behälter geformt ist, und die Kappe und der Behälter aus Kunststoff geformt sind.

20. Behälter nach Anspruch 19, umfassend eine Funkfrequenzkennungsmarke, die durch die Ansätze an ihrer Stelle zurückgehalten wird.

21. Behälter nach Anspruch 19, wobei der vertiefte Abschnitt durch einen Rand von einem unteren Ende des Behälters versetzt ist.

22. Behälter nach Anspruch 21, ferner umfassend eine Einkerbung, die sich im Rand befindet.

23. Behälter nach Anspruch 1, wobei der Behälter und die Kappe manipulationssicher sind, um anzuzeigen, ob der Behälter geöffnet worden ist, umfassend
einen zerstörbaren Vorsprung, der sich von einem aus dem Behälter und der Kappe zum anderen erstreckt,
wobei der Vorsprung einen Schaft beinhaltet, der einstückig mit einem aus dem Behälter und der Kappe geformt ist, und an einem freien Ende des Schafts zumindest einen sich seitlich erstreckenden Flansch beinhaltet, wobei der Schaft einen einzelnen geschwächten Punkt mit einem verringerten Querschnitt beinhaltet, der an einem Ende des Schafts gegenüber seinem freien Ende angeordnet ist, wobei sich ein Querstück an einer Stelle zwischen dem freien Ende und dem geschwächten Punkt seitlich vom Schaft erstreckt,
wobei das Querstück koplanar mit dem Schaft und dem Flansch ist,
einen länglichen Schlitz, der im anderen aus dem Behälter und der Kappe ausgebildet ist und das freie Ende des Vorsprungs aufnimmt, wobei sich der Flansch seitlich über einen Rand des Schlitzes hinaus erstreckt, so dass die Kappe nur als Reaktion auf einen Bruch des Vorsprungs am geschwächten Punkt geöffnet werden kann, wobei sich der Schlitz zwischen dem Flansch und dem Querstück befindet, um eine Trennung des Vorsprungs vom anderen aus dem Behälter und der Kappe zu verhindern, nachdem der Vorsprung zerbrochen wurde,
wobei der Behälter und die Kappe ein zweites zerstörbares Element beinhalten, das zu einem Einwegdurchgang durch eine Öffnung im anderen aus dem Behälter und der Kappe geeignet ist.

24. Behälter nach Anspruch 23, umfassend eine Funkfrequenzkennungsmarke, die durch die Ansätze an ihrer Stelle zurückgehalten wird.

25. Behälter nach Anspruch 23, wobei der vertiefte Abschnitt durch einen Rand von einem unteren Ende des Behälters versetzt ist.

26. Behälter nach Anspruch 25, ferner umfassend eine Einkerbung, die sich im Rand befindet.

27. Behälter nach Anspruch 1, wobei der Behälter und die Kappe manipulationssicher sind,
wobei der Behälter einen ersten Flansch beinhaltet, der eine erste Durchgangsöffnung aufweist, wobei die Kappe einen zweiten Flansch beinhaltet, der eine zweite Durchgangsöffnung aufweist,
wobei der zweite Flansch so angeordnet ist, dass er im Wesentlichen über dem ersten Flansch liegt, wenn die Kappe auf einen Rand des Behälters eingesetzt ist,
wobei eines aus dem Behälter und der Kappe einen einstückigen Streifen beinhaltet, der sich von einer Stelle neben den Durchgangslöchern erstreckt, wobei der Streifen eine ausreichende Biegsamkeit und Länge aufweist, um zu ermöglichen, dass der Streifen durch die Durchgangsöffnungen eingesetzt wird, während er am einen aus dem Behälter und der Kappe angebracht verbleibt,
wobei der Streifen Abschnitte beinhaltet, die für einen Einwegdurchgang durch die Durchgangsöffnungen gestaltet sind,
wobei der Behälter ferner mit einem vertieften Abschnitt versehen ist, der sich an einer Unterseite des Behälters befindet, wobei der vertiefte Abschnitt Ansätze aufweist, die in eine Rückhaltestellung fixiert werden können, um eine Funkfrequenzkennungsmarke an ihrer Stelle zurückzuhalten.

28. Behälter nach Anspruch 27, umfassend eine Funkfrequenzkennungsmarke, die durch die Ansätze an ihrer Stelle zurückgehalten wird.

29. Behälter nach Anspruch 27, wobei der vertiefte Abschnitt durch einen Rand von einem unteren Ende des Behälters versetzt ist.

30. Behälter nach Anspruch 29, ferner umfassend eine Einkerbung, die sich im Rand befindet.

31. Behälter nach Anspruch 1, wobei der Behälter und die Kappe manipulationssicher sind, umfassend einen Behälter und eine Kappe aus einem einstückig geformten einteiligen Aufbau, wobei dazwischen ein Gelenk beinhaltet ist, wobei der Behälter einen ersten Flansch beinhaltet, der eine erste Durchgangsöffnung aufweist, wobei die Kappe einen zweiten Flansch beinhaltet, der so angeordnet ist, dass er über dem ersten Flansch liegt, wenn die Kappe auf einen Rand des Behälters eingesetzt ist, wobei der Flansch von einem des Behälters und der Kappe einen Streifen beinhaltet, der damit einstückig in einem Teil geformt ist, wobei der Streifen ausreichend biegsam und von einer Länge ist, die ausreicht, um zu ermöglichen, dass der Streifen durch die Öffnungen eingesetzt wird, während er am letztgenannten Flansch angebracht verbleibt, wobei der Streifen Abschnitte beinhaltet, die für einen Einwegdurchgang durch die Durchgangsöffnungen gestaltet sind, wobei eine Entfernung des Streifens nur durch seine dauerhafte Zerstörung möglich ist, wobei der zweite Flansch eine Daumenlasche definiert, die das Öffnen der Kappe erleichtert.

32. Behälter nach Anspruch 31, umfassend eine Funkfrequenzkennungsmarke, die durch die Ansätze an ihrer Stelle zurückgehalten wird.

33. Behälter nach Anspruch 31, wobei der vertiefte Abschnitt durch einen Rand von einem unteren Ende des Behälters versetzt ist.

34. Behälter nach Anspruch 33, ferner umfassend eine Einkerbung, die sich im Rand befindet.

## Revendications

1. Récipient comprenant :
un flacon jetable en matière plastique ;
un bouchon ;
une partie en retrait située sur un côté de fond du récipient (10) ;
dans lequel la partie en retrait comporte des pattes (20) qui peuvent être fixées dans une position de retenue afin de retenir en place une étiquette d'identification par radio fréquence (RFID) ; et dans lequel
lesdites pattes peuvent être assouplies sous l'action d'une source de chaleur et sont fixées dans la position de retenue sous l'action d'une pression dans l'état d'assouplissement.

2. Récipient selon la revendication 1, comprenant en outre une encoche (24) située sur le côté de fond.

3. Récipient selon la revendication 1 comprenant une étiquette RFID retenue en place par les pattes.

4. Récipient selon la revendication 1 dans lequel la partie en retrait est décalée d'une extrémité de fond du récipient par un rebord (17).

5. Récipient selon la revendication 4, comprenant en outre une encoche (24) située dans le rebord (17).

6. Récipient comprenant une surface de fond en retrait d'une extrémité de fond d'une paroi latérale de récipient, la surface de fond se composant :
d'une bague (18) suspendue à la surface de fond dans une direction en retrait par rapport à la surface de fond sur une première dimension de hauteur ;
d'une pluralité de pattes (20) qui s'étendent à partir de la bague dans une direction davantage en retrait de la surface de fond jusqu'à une seconde dimension de hauteur qui est plus élevée que la première dimension de hauteur ;
d'une pluralité d'entretoises (22) positionnées sur un côté intérieur de la bague ;
dans lequel un espace est défini à l'intérieur des entretoises dans lequel il est possible de positionner une étiquette RFID, et dans lequel il est possible de fixer les pattes dans une position dans laquelle elles s'étendent par dessus l'étiquette lorsque l'étiquette est positionnée dans l'espace.

7. Récipient selon la revendication 6 dans lequel la surface de fond est décalée de l'extrémité de fond du récipient par un rebord (17).

8. Récipient selon la revendication 6, comprenant en outre une encoche (24) située dans le côté de fond.

9. Récipient selon la revendication 8, dans lequel l'encoche (24) est située dans le rebord (17).

10. Récipient selon la revendication 6 dans lequel le rebord (17) comporte une pluralité de nervures (50).

11. Récipient selon la revendication 1 dans lequel le récipient et le bouchon sont inviolables afin d'indiquer si le récipient a été ouvert, et comprennent :
une saillie destructible s'étendant entre l'un et l'autre dudit récipient et dudit bouchon, ladite saillie comprenant une tige moulée intégralement avec l'un dudit récipient et dudit bouchon et au moins une bride qui s'étend latéralement à une extrémité libre de ladite tige, ladite tige comprenant un seul point faible ayant une coupe transversale réduite situé à une extrémité de ladite tige en opposition à ladite extrémité libre de celle-ci, une pièce transversale étant en saillie étant en saillie latéralement à partir de ladite tige une position intermédiaire entre ladite extrémité libre et ledit point faible, ladite pièce transversale étant coplanaire avec ladite tige et ladite bride,
une fente allongée formée dans l'autre dudit récipient et dudit bouchon et recevant ladite extrémité libre de ladite saillie, ladite bride s'étendant latéralement au-delà d'un bord de ladite fente de telle sorte que ledit bouchon peut être ouvert uniquement en réponse à une rupture de ladite saillie audit point faible, ladite fente étant située entre ladite bride et la pièce transversale afin d'empêcher une séparation de ladite saillie de l'autre dudit récipient et dudit bouchon une fois que ladite saillie a été brisée, ledit récipient comprenant un canal qui s'étend dans le sens de la circonférence autour d'une surface externe de celui-ci, ledit canal étant ouvert vers ledit bouchon, ledit canal recevant un bord périphérique dudit bouchon lorsque ledit bouchon est fermé.

12. Récipient selon la revendication 11 comprenant une étiquette RFID retenue en place par les pattes.

13. Récipient selon la revendication 11 dans lequel la partie en retrait est décalée d'une extrémité de fond du récipient par un rebord.

14. Récipient selon la revendication 12, comprenant en outre une encoche située dans le rebord.

15. Récipient selon la revendication 1 dans lequel le récipient et le bouchon sont inviolables afin d'indiquer si le récipient a été ouvert, et comprenant :
une connexion destructible disposée entre ledit récipient et ledit bouchon comprenant un élément destructible qui maintien ledit bouchon dans une position fermée de telle sorte que ledit bouchon peut être ouvert uniquement en réponse à la destruction dudit élément destructible, ledit récipient comprenant un rebord annulaire formant une ouverture dudit récipient, et un canal annulaire ouvert vers le haut s'étendant dans le sens de la circonférence autour d'une surface externe dudit récipient à un emplacement situé en dessous dudit rebord,
ledit bouchon comprenant :
un retrait destiné à recevoir ledit rebord afin de fermer ladite ouverture ;
un bord périphérique reçu dans ledit canal lorsque ledit bouchon est fermé ; et
un élément d'étanchéité annulaire disposé à l'intérieur dudit retrait à un emplacement situé au-dessus dudit bord périphérique, ledit élément d'étanchéité annulaire formant un espace ouvert vers le bas dans lequel est reçu ledit rebord afin de former un joint d'étanchéité.

16. Récipient selon la revendication 15 comprenant une étiquette RFID maintenue en place par les pattes.

17. Récipient selon la revendication 15 dans lequel la partie en retrait est décalée d'une extrémité de fond du récipient par un rebord.

18. Récipient selon la revendication 17, comprenant en outre une encoche située dans le rebord.

19. Récipient selon la revendication 1 dans lequel le récipient et le bouchon sont inviolables afin d'indiquer si le récipient a été ouvert, et comprennent :
une saillie destructible s'étendant entre l'un et l'autre dudit récipient et dudit bouchon, ladite saillie comprenant une tige moulée intégralement avec l'un dudit récipient et dudit bouchon et au moins une bride qui s'étend latéralement à une extrémité libre de ladite tige, ladite tige comprenant un seul point faible ayant une coupe transversale réduite situé à une extrémité de ladite tige en opposition à ladite extrémité libre de celle-ci, une pièce transversale étant en saillie latéralement à partir de ladite tige dans une position intermédiaire entre ladite extrémité libre et ledit point faible, ladite pièce transversale étant coplanaire avec ladite tige et ladite bride,
une fente allongée formée dans l'autre dudit récipient et dudit bouchon et recevant l'extrémité libre de ladite saillie, ladite bride s'étendant latéralement au-delà d'un bord de ladite fente de telle sorte que ledit bouchon peut être ouvert uniquement en réponse à une rupture de ladite saillie audit point faible, ladite fente étant située entre ladite bride et la pièce transversale afin d'empêcher une séparation de ladite saillie de l'autre dudit récipient et dudit bouchon une fois que ladite saillie a été brisée, ledit récipient étant moulé en une seule pièce avec ledit récipient, et ledit bouchon et ledit récipient étant moulés dans une matière plastique.

20. Récipient selon la revendication 19 comprenant une étiquette RFID retenue en place par les pattes.

21. Récipient selon la revendication 19 dans lequel la partie en retrait est décalée d'une extrémité de fond du récipient par un rebord.

22. Récipient selon la revendication 21, comprenant en outre une encoche située dans le rebord.

23. Récipient selon la revendication 1 dans lequel le récipient et le bouchon sont inviolables afin d'indiquer si le récipient a été ouvert, et comprenant :
une saillie destructible s'étendant entre l'un et l'autre dudit récipient et dudit bouchon, ladite saillie comprenant une tige moulée intégralement avec l'un dudit récipient et dudit bouchon et au moins une bride qui s'étend latéralement à une extrémité libre de ladite tige, ladite tige comprenant un seul point faible ayant une coupe transversale réduite situé à une extrémité de ladite tige en opposition à ladite extrémité libre de celle-ci, une pièce transversale étant en saillie latéralement à partir de ladite tige à un emplacement intermédiaire situé entre ladite extrémité libre et ledit point faible, ladite pièce transversale étant coplanaire avec ladite tige et ladite bride,
une fente allongée formée dans l'autre dudit récipient et dudit bouchon et recevant ladite extrémité libre de ladite saillie, ladite bride s'étendant latéralement au-delà d'un bord de ladite fente de telle sorte que ledit bouchon peut être ouvert uniquement en réponse à une rupture de ladite saillie audit point faible, ladite fente étant située entre ladite bride et la pièce transversale afin d'empêcher une séparation de ladite saillie de l'autre dudit récipient et dudit bouchon une fois que ladite saillie a été brisée, ledit récipient et ledit bouchon comprenant un second élément destructible adapté pour un passage unidirectionnel au travers d'un trou aménagé dans un autre dudit récipient et dudit bouchon.

24. Récipient selon la revendication 23 comprenant une étiquette RFID retenue en place par les pattes.

25. Récipient selon la revendication 23 dans lequel la partie en retrait est décalée d'une extrémité de fond du récipient par un rebord.

26. Récipient selon la revendication 25, comprenant en outre une encoche située dans le rebord.

27. Récipient selon la revendication 1 dans lequel le récipient et le bouchon sont inviolables, ledit récipient comprenant une première bride comportant un premier trou traversant, ledit bouchon comprenant une seconde bride comportant un second trou traversant, ladite seconde bride étant aménagée de manière à recouvrir essentiellement ladite première bride lorsque ledit bouchon est introduit sur un rebord dudit récipient, l'un dudit récipient et dudit bouchon comprenant une bande incorporée qui s'étend à partir d'un emplacement situé à proximité desdits trous traversants, ladite bande ayant une souplesse et une longueur suffisantes pour permettre l'introduction de ladite bande au travers desdits trous traversants tout en restant fixée sur l'un dudit récipient et dudit bouchon, ladite bande comprenant des parties configurées pour un passage unidirectionnel au travers desdits trous traversants,
dans lequel le récipient comporte en outre une partie en retrait située sur un côté de fond du récipient, la partie en retrait comportant des pattes qui peuvent être fixées dans une position de retenue afin de retenir en place l'étiquette RFID.

28. Récipient selon la revendication 27 comprenant une étiquette RFID retenue en place par les pattes.

29. Récipient selon la revendication 27 dans lequel la partie en retrait est décalée d'une extrémité de fond du récipient par un rebord.

30. Récipient selon la revendication 29, comprenant en outre une encoche située dans le rebord.

31. Récipient selon la revendication 1 dans lequel le récipient et le bouchon sont inviolables et se composent d'un récipient et d'un bouchon fabriqués par moulage en une seule pièce et qui comprennent entre eux une articulation, ledit récipient comprenant une première bride comportant un premier trou traversant, ledit bouchon comprenant une seconde bride aménagée de manière à recouvrir ladite première bride lorsque ledit bouchon est introduit sur un rebord dudit récipient, ladite bride de l'un dudit récipient et dudit bouchon comprenant une bande intégralement moulée en une seule pièce avec ceux-ci, ladite bride étant suffisamment souple et ayant une longueur suffisante pour permettre l'introduction de ladite bande au travers desdits trous traversants tout en restant à ladite bride nommée en dernier, ladite bande comprenant des parties configurées pour un passage unidirectionnel au travers desdits trous traversants, ce qui permet l'enlèvement de ladite bande uniquement par une destruction définitive de celle-ci, ladite seconde bride définissant une languette à tirage manuel qui facilite l'ouverture dudit bouchon.

32. Récipient selon la revendication 31 comprenant une étiquette RFID retenue en place par les pattes.

33. Récipient selon la revendication 31 dans lequel la partie en retrait est décalée d'une extrémité de fond du récipient par un rebord.

34. Récipient selon la revendication 33, comprenant en outre une encoche située dans le rebord.
